# EUROPEAN PATENT APPLICATION

(11) **EP 4 599 854 A1**
(43) Date of publication of application: **13.08.2025**
(21) Application number: 24832188.7
(22) Date of filing: 02.04.2024
(51) Int. Cl.: A61L 2/10, H01J 61/073, H01J 61/02, H01J 61/36, A61L 9/20

(54) **FAR ULTRAVIOLET RAY-EMITTING DEVICE**

(30) Priority: 30.06.2023 KR 20230085100; 10.07.2023 KR 20230089040
(71) Applicant: LG Electronics Inc., Yeongdeungpo-gu Seoul 07336 (KR)
(72) Inventor: OH, Jinmok, Seoul 08592 (KR); PARK, Deokhai, Seoul 08592 (KR); NAMKUNG, Seok, Seoul 08592 (KR); KANG, Namseok, Seoul 08592 (KR)
(74) Representative: Vossius & Partner Patentanwälte Rechtsanwälte mbB
(86) International application number: PCT/KR2024/004233
(87) International publication number: WO 2025/005403

(57) **Abstract**

A far ultraviolet light emitting device may include a surface light source configured to emit light by a surface discharge; and a bandpass filter positioned on a light emitting surface of the surface light source. The surface light source may include a first plate member including the light emitting surface; a second plate member configured to face the first plate member with a discharge space interposed therebetween, the second plate member including an outer surface including an electrode surface; and a first electrode and a second electrode positioned on the electrode surface of the second plate member. According to this configuration, the first electrode and the second electrode are disposed only on the electrode surface of the second plate member and are not disposed on the first plate member. Therefore, the surface light source emits light by the surface charge not a dielectric barrier discharge and can sufficiently secure an acceleration distance of electrons or ions that generate discharge, thereby improving luminous efficiency. In addition, since the surface light source can reduce the material cost of the electrodes, the manufacturing cost of the far ultraviolet light emitting device can be reduced.

## Description

### [Technical Field]

The present disclosure relates to a far ultraviolet light emitting device generating ultraviolet rays harmless to a human body.

### [Background Art]

In general, ultraviolet lamps are used in various fields to sterilize bacteria and fungi by generating ultraviolet rays.

The ultraviolet lamp can be suitably used with easy operation at the necessary time by using a lamp method. The installation cost and the maintenance cost for installing the ultraviolet lamp are low. In addition, the ultraviolet rays generated by the ultraviolet lamp rarely change and thus continuously have the same sterilizing power.

The ultraviolet lamp generates ultraviolet (UV) rays with various wavelengths based on a material provided inside the lamp. For example, UV-A (315 nm to 400 nm), UV-B (280 nm to 315 nm), UV-C (100 nm to 280 nm), and the like may be generated.

Among them, ultraviolet rays with a wavelength corresponding to UV-C have the best sterilizing power. When UV-C is irradiated to bacteria, fungi, etc., bacteria and fungi die because their DNA is damaged. That is, ultraviolet rays have an effective sterilizing power against various bacteria by damaging DNA of living organisms.

Therefore, sterilization using the ultraviolet lamp is more efficient than sterilization using heat, chemicals, ozone, or radiation.

The ultraviolet lamp, particularly an excimer lamp, may be generally classified into a tube type lamp and a plate type lamp.

A tube included in the tube type lamp may be classified into a circular tube having a substantially circular cross-sectional shape and a rectangular tube having a substantially rectangular cross-sectional shape, and the rectangular tube may be manufactured by forming the circular tube using a processing method such as thermoforming.

The plate type lamp may be formed by sealing two plates with a sealant.

Each of the tube included in the tube type lamp and the plate included in the plate type lamp may be formed of quartz.

An example of an ultraviolet irradiation device provided with the tube type lamp including the circular tube is disclosed in Korean Patent Application Publication No. 10-2021-0049890 (hereinafter, referred to as "prior patent 1"), and an example of the excimer lamp provided with the tube type lamp including the rectangular tube is disclosed in Korean Patent No. 10-1646862 (hereinafter referred to as "prior patent 2").

The ultraviolet irradiation device of the prior patent 1 includes a lamp house having a light extraction surface formed on at least one surface, a plurality of excimer lamps that are positioned to be spaced apart from the light extraction surface in a first direction within the lamp house and generate ultraviolet rays whose main emission wavelength is included in a first wavelength range (190 nm or more and 225 nm or less), a first electrode disposed in contact with an outer surface of the excimer lamp, a second electrode that is disposed in contact with the outer surface of the excimer lamp at a position spaced apart from the first electrode in a second direction parallel to a tube axis of the excimer lamp, and an optical filter that is disposed on the light extraction surface and substantially transmits ultraviolet rays of the first wavelength range while substantially blocking ultraviolet rays in a wavelength range (240 nm or more and 300 nm or less) harmful to the human body.

However, in the ultraviolet irradiation device of the prior patent 1, since a tube included in the excimer lamp is formed in a circular shape, the plurality of excimer lamps must be disposed in the ultraviolet irradiation device in order to form a surface light source structure.

Here, a "surface light source" refers to a light source in which most of a surface from which light is emitted, for example, a light emitting surface, is substantially flat.

Therefore, when the tube included the excimer lamp is formed in the circular shape, the surface light source structure must be formed using the plurality of excimer lamps.

However, each of the lamp having the rectangular tube and the plate type lamp may form the surface light source structure with one or more lamps.

Therefore, the ultraviolet irradiation device of the prior patent 1 has a problem in that the number of lamps increases compared to the lamp having the rectangular tube and the plate type lamp, which increases the manufacturing cost.

Further, in forming the surface light source structure using the plurality of excimer lamps, since the distance between the excimer lamps adjacent to each other along the first direction needs to be kept at a constant interval, there is a problem in that the amount of light is small compared to the area of the lamp housing. That is, there is a problem in that the area of the lamp housing is large compared to the amount of light.

The prior patent 2 discloses the excimer lamp including the tube type lamp formed of the rectangular tube.

The rectangular tube of the prior patent 2 includes a pair of flat wall portions and a side wall portion connecting the flat wall portions, and the flat wall portions form a light emitting surface.

As described above, the excimer lamp of the prior patent 2 may be considered to have a surface light source structure because the flat wall portion of the rectangular tube forms the light emitting surface.

However, since the excimer lamp of the prior patent 2 is formed only with heat, it is difficult to form a surface light source with precise dimensions. Further, since both ends of the surface light source have to be joined using a sealant, there is a problem in that the manufacturing process is complicated.

In addition, electrodes with different polarities are disposed opposite to each other on the pair of flat wall portions included in the rectangular tube of the prior patent 2.

That is, the first electrode or the second electrode is disposed on one flat wall portion, and the other electrode, for example, the second electrode or the first electrode, is disposed on the other flat wall portion.

Therefore, in the excimer lamp of the prior patent 2 in which the first electrode and the second electrode with different polarities are disposed opposite to each other on different flat wall portions, since light emission is generated by dielectric barrier discharge (DBD), there is a problem in that a sufficient luminous efficiency cannot be obtained due to a short acceleration distance of electrons and ions that generate discharge.

Further, since the first electrode and the second electrode are respectively disposed on the flat wall portions, an area of a light emitting surface from which light is emitted through the flat wall portion decreases due to the electrode. Therefore, there is a problem in that the light extraction efficiency is low.

Further, in the excimer lamp of the prior art 2, the electrode positioned on the light emitting surface should be formed into a mesh type in order to secure the light emitting surface. In order to form the mesh type electrode, an electrode layer should be formed in the entire area of the light emitting surface, and then a portion of the electrode layer should be removed to form the mesh type electrode.

Therefore, there is a problem in that the material cost of the electrode is high due to the electrode layer that cannot be used as the electrode and is removed.

Further, in the excimer lamp of the prior patent 2, since an ultraviolet reflective layer for reflecting ultraviolet rays is disposed on an inner surface of the flat wall portion, it is difficult to form the ultraviolet reflective layer.

As a light emitting device including the plate type lamp, there is disclosed an ultraviolet irradiation device in which a spacer is disposed between two plates to form a discharge space and then the two plates are sealed with a sealant, or one surface of a plate formed to be relatively thick among the two plates is etched to form the discharge space and then the two plates are sealed with the sealant.

The far ultraviolet light emitting device including the plate type lamp can be considered to have the surface light source structure since the plate forms the light emitting surface.

However, the far ultraviolet light emitting device including the plate type lamp contains the same problem that occurs in the prior patent 2, because the first electrode and the second electrode with different polarities are disposed opposite to each other on different plates in the same manner as the prior patent 2.

Further, in the far ultraviolet light emitting device including the plate type lamp formed by sealing two plates, since it requires a sealant for joining the two plates and an exhaust tip for exhausting and injecting gas, there is a problem in that the manufacturing process of the far ultraviolet light emitting device is complicated.

In the existing far ultraviolet light emitting devices disclosed in the prior patents 1 and 2, after a bandpass filter for blocking ultraviolet rays with wavelengths harmful to humans is coated on a separate quartz plate (e.g., a fused quartz substrate), the fused quartz substrate coated with the bandpass filter is provided on a light extraction surface. Therefore, there is a problem in that a light transmittance decreases due to the use of the fused quartz substrate.

That is, when the fused quartz substrate for installing the bandpass filter is provided, a portion of light is reflected from a boundary between the lamp and the fused quartz substrate and cannot be emitted to the outside of the lamp, which causes a problem in that the light transmittance decreases.

### [Prior Art Document]

### [Patent Document]

Prior Patent 1: Korean Patent Application Publication No. 10-2021-0049890
Prior Patent 2: Korean Patent No. 10-1646862

### [Disclosure]

### [Technical Problem]

A technical object of the present disclosure is to provide a far ultraviolet light emitting device capable of solving at least one of the above-described problems.

Another technical object of the present disclosure is to provide a far ultraviolet light emitting device including a surface light source that emits light by a surface discharge.

Another technical object of the present disclosure is to provide a far ultraviolet light emitting device including a surface light source capable of sufficiently securing an acceleration distance of electrons or ions that generate a discharge.

Another technical object of the present disclosure is to provide a far ultraviolet light emitting device including a surface light source with improved luminous efficiency.

Another technical object of the present disclosure is to provide a far ultraviolet light emitting device including a surface light source with improved light extraction efficiency.

Another technical object of the present disclosure is to provide a far ultraviolet light emitting device including a surface light source in which electrodes with different polarities are disposed on the same surface.

Another technical object of the present disclosure is to provide a far ultraviolet light emitting device in which a bandpass filter is disposed on a light emitting surface of a surface light source.

Another technical object of the present disclosure is to provide a far ultraviolet light emitting device including a surface light source with precise dimensions.

Another technical object of the present disclosure is to provide a far ultraviolet light emitting device including a surface light source with a simple manufacturing process.

The technical objects to be achieved by the present disclosure are not limited to those that have been described hereinabove merely by way of example, and other technical objects that are not mentioned can be clearly understood by those skilled in the art, to which the present disclosure pertains, from the following descriptions.

### [Technical Solution]

In one aspect of the present disclosure, there is provided a far ultraviolet light emitting device comprising a surface light source configured to emit light by a surface discharge; and a bandpass filter positioned on a light emitting surface of the surface light source.

The surface light source of the far ultraviolet light emitting device according to the aspect of the present disclosure may include a first plate member including the light emitting surface; a second plate member configured to face the first plate member with a discharge space interposed therebetween, the second plate member including an outer surface including an electrode surface; and a first electrode and a second electrode positioned on the electrode surface of the second plate member.

According to the above configuration, the first electrode and the second electrode are disposed only on the electrode surface of the second plate member and are not disposed on the first plate member.

Therefore, the surface light source emits light by the surface charge not a dielectric barrier discharge and can sufficiently secure an acceleration distance of electrons or ions that generate discharge, thereby improving luminous efficiency.

In addition, since the surface light source can reduce the material cost of the electrodes, the manufacturing cost of the far ultraviolet light emitting device can be reduced.

The first electrode may include a first main electrode portion, and the second electrode may include a second main electrode portion. The first main electrode portion and the second main electrode portion may be positioned to be spaced apart from each other at a first interval.

According to the above configuration, it is possible to appropriately control the acceleration distance of electrons or ions by appropriately setting a size of the first interval.

The first electrode and the second electrode may be formed in an asymmetrical structure or a symmetrical structure.

When the first electrode and the second electrode are formed in the asymmetrical structure, one of the first electrode and the second electrode may include at least one auxiliary electrode portion protruding toward the main electrode portion of the other electrode.

When the auxiliary electrode portion is formed, a discharge inception voltage can be reduced.

When the first electrode and the second electrode are formed in the symmetrical structure, the far ultraviolet light emitting device may further comprise an auxiliary electrode positioned between the first main electrode portion and the second main electrode portion.

When the auxiliary electrode is formed, the discharge inception voltage can be reduced.

When the first electrode and the second electrode are formed in the symmetrical structure, the first electrode may further include at least one first auxiliary electrode portion protruding toward the second main electrode portion, and the second electrode may further include at least one second auxiliary electrode portion protruding toward the first main electrode portion.

The at least one first auxiliary electrode portion and the at least one second auxiliary electrode portion may face each other.

When the first auxiliary electrode portion and the second auxiliary electrode portion are formed, the discharge inception voltage can be further reduced.

The first plate member and the second plate member may be made of quartz glass or ceramic.

The second plate member may include a concave portion for forming the discharge space and a first wall member that is positioned at an edge of the second plate member and surrounds the concave portion. A sealant may be positioned between the first wall member and the first plate member.

Therefore, since the second plate member including the first wall member is attached to the first plate member by the sealant, the discharge space can be maintained in a sealed state.

The second plate member may further include a second wall member positioned in the discharge space.

According to the above configuration, a distance between the first plate member and the second plate member can be uniformly maintained by the first wall member and the second wall member.

The bandpass filter may be positioned on a fused silica substrate, and the fused silica substrate including the bandpass filter may be positioned on the light emitting surface of the first plate member.

According to the above configuration, ultraviolet rays in a wavelength range harmful to the human body can be blocked.

Alternatively, the bandpass filter may be directly coated on the light emitting surface of the first plate member.

According to the above configuration, a reflection of some light at a boundary between the first plate member and the fused silica substrate can be reduced, and thus light extraction efficiency can be increased.

Further, the manufacturing cost of the far ultraviolet light emitting device can be reduced because the fused silica substrate is not used.

The discharge space may include an inert gas selected from the group consisting of argon (Ar), neon (Ne), xenon (Xe), and krypton (Kr), and the inert gas may be selected from the group consisting of ArBr, ArCl, ArF, ArO, NeF, XeI, XeO, XeBr, XeCl, XeF, KrBr, KrCl, KrO, and KrF.

Instead of the second plate member including the wall member, the surface light source may further include a first spacer positioned between an edge of the first plate member and an edge of the second plate member. A sealant may be positioned between the first spacer and the first plate member and between the first spacer and the second plate member.

Since the first spacer is joined to each of the first plate member and the second plate member, the discharge space can be maintained in a sealed state.

The surface light source may further include a second spacer positioned in the discharge space.

According to the above configuration, a distance between the first plate member and the second plate member can be uniformly maintained by the first spacer and the second spacer.

A surface light source of a far ultraviolet light emitting device according to another aspect of the present disclosure may include a light emitting portion including the light emitting surface; an electrode portion configured to face the light emitting portion with a discharge space interposed therebetween, the electrode portion including an electrode surface on which a first electrode and a second electrode are disposed; and a side wall portion configured to connect the light emitting portion and the electrode portion along a first direction. A first end and a second end may be positioned on both sides of the surface light source in the first direction. The first end may be formed as a joined end at which the light emitting portion and the electrode portion are joined in direct contact with each other, and the second end may be formed as a sealed end that extends from the light emitting portion and the electrode portion in the first direction and is sealed by a sealant.

The surface light source of the above-described configuration may be formed by heating a cylindrical tube and molding the cylindrical tube at the outside of the cylindrical tube using a mold.

The light emitting portion, the electrode portion, and the joined end of the surface light source may be formed during a molding operation using the mold.

The light emitting portion and the electrode portion may have a uniform thickness by injecting a compressed air into the cylindrical tube during the molding operation using the mold.

According to the above configuration, it is possible to form the surface light source with precise dimensions.

The joined end may be formed in a shape with a flat rectangular cross-section, and the sealed end may be formed in a shape with a circular cross-section.

According to the above configuration, an unprocessed portion of the cylindrical tube may be used as the sealed end, and after exhaust and gas injection are performed through the sealed end, the sealed end may be sealed using a sealant.

Therefore, there is no need to form an exhaust tip separately.

The light emitting portion and the electrode portion may be in direct contact with each other at the joined end.

A thickness of the joined end may be less than a thickness of the surface light source, and a thickness of the sealed end may be greater than the thickness of the surface light source.

That is, since the cross-sectional shape of the sealed end is a circle, the exhaust and gas injection can be performed smoothly.

Each of the light emitting portion and the electrode portion may be substantially flat formed during the molding operation using the mold.

Alternatively, at least one of the light emitting portion and the electrode portion may be formed to have at least one bent portion during the molding operation using the mold.

According to the above configuration, since the number of discharge filaments with a high plasma density can be increased, an amount of ultraviolet rays can be increased, and stable discharge can be maintained.

The first electrode and the second electrode are disposed only on the electrode surface of the electrode portion and are not disposed on the light emitting surface of the light emitting portion.

Therefore, the surface light source emits light by the surface charge not the dielectric barrier discharge and can sufficiently secure an acceleration distance of electrons or ions that generate discharge, thereby improving luminous efficiency.

In addition, since the surface light source can reduce the material cost of the electrodes, the manufacturing cost of the far ultraviolet light emitting device can be reduced.

The first electrode may include the first main electrode portion, and the second electrode may include the second main electrode portion. The first main electrode portion and the second main electrode portion may be positioned to be spaced apart from each other at the first interval.

According to the above configuration, it is possible to appropriately control the acceleration distance of electrons or ions by appropriately setting the size of the first interval.

Each of the first electrode and the second electrode may be coated, printed, or deposited on the surface of the electrode portion, and may be in direct contact with the surface of the electrode portion.

According to the above configuration, after the cylindrical tube is formed, the first electrode and the second electrode may be formed directly on the surface of the electrode portion.

Alternatively, each of the first electrode and the second electrode may be formed as a metal thin plate and may be in direct contact with the surface of the electrode portion.

According to the above configuration, the first electrode and the second electrode may be disposed directly on the electrode portion during the molding operation using the mold.

In this case, a portion of each of the first electrode and the second electrode may be embedded in the electrode portion.

According to the above configuration, the first electrode and the second electrode can be firmly fixed to the electrode portion.

The surface light source may further include a reflective member between the first electrode and the electrode portion and between the second electrode and the electrode portion.

The reflective member may be formed of a PTFE material which is one of fluorine-based polymers with frail properties.

According to the above configuration, the electrodes can be firmly attached to the reflective member almost without being affected by the thickness non-uniformity of the electrode portion and the thickness non-uniformity of the electrode material.

By arranging the reflective member formed of a material having a higher reflectance than the electrode material between the electrodes and the electrode portion, a loss of ultraviolet rays emitted from the surface light source can be minimized, and the freedom of design of the electrode structure or the surface light source can be improved.

Each of the first electrode and the second electrode may be coated, printed, or deposited on the reflective member, and may be in direct contact with the surface of the reflective member.

According to the above configuration, after the cylindrical tube is formed, the reflective member may be formed directly on the surface of the electrode portion, and then the first electrode and the second electrode may be formed directly on the surface of the reflective member.

Alternatively, each of the first electrode and the second electrode may be formed as a metal thin plate and may be in direct contact with the surface of the reflective member.

According to the above configuration, the reflective member may be disposed directly on the electrode portion during the molding operation using the mold.

In this case, a portion of the reflective member may be embedded in the electrode portion.

According to the above configuration, the reflective member can be firmly fixed to the electrode portion.

The first electrode and the second electrode may be formed in an asymmetrical structure or a symmetrical structure.

When the first electrode and the second electrode are formed in the asymmetrical structure, one of the first electrode and the second electrode may include at least one auxiliary electrode portion protruding toward the main electrode portion of the other electrode.

When the auxiliary electrode portion is formed, the discharge inception voltage can be reduced.

When the first electrode and the second electrode are formed in the symmetrical structure, the far ultraviolet light emitting device may further comprise an auxiliary electrode positioned between the first main electrode portion and the second main electrode portion.

When the auxiliary electrode is formed, the discharge inception voltage can be reduced.

When the first electrode and the second electrode are formed in the symmetrical structure, the first electrode may further include at least one first auxiliary electrode portion protruding toward the second main electrode portion, and the second electrode may further include at least one second auxiliary electrode portion protruding toward the first main electrode portion.

The at least one first auxiliary electrode portion and the at least one second auxiliary electrode portion may face each other.

When the first auxiliary electrode portion and the second auxiliary electrode portion are formed, the discharge inception voltage can be further reduced.

The light emitting portion, the electrode portion, the joined end, and the sealed end may be made of quartz glass or ceramic.

The bandpass filter may be positioned on the fused silica substrate, and the fused silica substrate including the bandpass filter may be positioned on the light emitting surface of the light emitting portion.

According to the above configuration, ultraviolet rays in a wavelength range harmful to the human body can be blocked.

Alternatively, the bandpass filter may be directly coated on the light emitting surface of the light emitting portion.

According to the above configuration, a reflection of some light at a boundary between the light emitting portion and the fused silica substrate can be reduced, and thus light extraction efficiency can be increased.

Further, the manufacturing cost of the far ultraviolet light emitting device can be reduced because the fused silica substrate is not used.

The discharge space formed between the light emitting portion and the electrode portion may include an inert gas selected from the group consisting of argon (Ar), neon (Ne), xenon (Xe), and krypton (Kr), and the inert gas may be selected from the group consisting of ArBr, ArCl, ArF, ArO, NeF, XeI, XeO, XeBr, XeCl, XeF, KrBr, KrCl, KrO, and KrF.

### [Advantageous Effects]

A surface light source included in a far ultraviolet light emitting device according to the present disclosure emits light by surface discharge not dielectric barrier discharge.

Therefore, an acceleration distance of electrons or ions that generate discharge can be sufficiently secured, and thus luminous efficiency is improved.

In addition, since the material cost of electrodes can be reduced, the manufacturing cost of the far ultraviolet light emitting device can be reduced.

Since at least one of a first electrode and a second electrode includes an auxiliary electrode portion, or an auxiliary electrode is formed between the first electrode and the second electrode, a discharge inception voltage is reduced by the auxiliary electrode portion or the auxiliary electrode.

Further, ultraviolet rays in a wavelength range harmful to the human body can be blocked using a bandpass filter.

Further, when the bandpass filter is directly coated on a light emitting surface of the surface light source, light extraction efficiency can be increased, and the manufacturing cost of the far ultraviolet light emitting device can be reduced because a fused silica substrate is not used.

Effects that could be achieved with the present disclosure are not limited to those that have been described hereinabove merely by way of example, and other effects and advantages of the present disclosure will be more clearly understood from the following description by a person skilled in the art to which the present disclosure pertains.

### [Description of Drawings]

The accompanying drawings, which are included to provide a further understanding of the present disclosure and constitute a part of the detailed description, illustrate embodiments of the present disclosure and serve to explain technical features of the present disclosure together with the description.
FIG. 1 is a perspective view of a surface light source according to a first embodiment of the present disclosure.
FIG. 2 is a cross-sectional view taken along "II-II" of FIG. 1.
FIG. 3 schematically illustrates an electrode structure included in the surface light source of FIG. 1.
FIG. 4 schematically illustrates a first modification of an electrode structure included in the surface light source of FIG. 1.
FIG. 5 schematically illustrates a second modification of an electrode structure included in the surface light source of FIG. 1.
FIG. 6 schematically illustrates a third modification of an electrode structure included in the surface light source of FIG. 1.
FIG. 7 schematically illustrates a fourth modification of an electrode structure included in the surface light source of FIG. 1.
FIG. 8 is a perspective view of a surface light source according to a second embodiment of the present disclosure.
FIG. 9 is a cross-sectional view taken along "IX-IX" of FIG. 8.
FIG. 10 is a table for measuring an amount of ultraviolet rays of a surface light source having an electrode structure according to a second modification of the present disclosure.
FIG. 11 is a plan view of a surface light source according to a third embodiment of the present disclosure.
FIG. 12 is a cross-sectional view taken along "XII-XII" of FIG. 11.
FIG. 13 is an enlarged view of main components of FIG. 12.
FIG. 14 schematically illustrates an electrode structure included in the surface light source of FIG. 11.
FIG. 15 schematically illustrates a first modification of an electrode structure included in the surface light source of FIG. 11.
FIG. 16 schematically illustrates a second modification of an electrode structure included in the surface light source of FIG. 11.
FIG. 17 schematically illustrates a third modification of an electrode structure included in the surface light source of FIG. 11.
FIG. 18 schematically illustrates a fourth modification of an electrode structure included in the surface light source of FIG. 11.
FIG. 19 is a plan view of a surface light source according to a fourth embodiment of the present disclosure.
FIG. 20 is a cross-sectional view taken along "XX-XX" of FIG. 19.
FIG. 21 is a cross-sectional view taken along "XXI-XXI" of FIG. 19.
FIG. 22 is a perspective view of a general mold used to manufacture a surface light source according to a third embodiment of the present disclosure.
FIG. 23 is a perspective view of a special mold used to manufacture a surface light source according to a fourth embodiment of the present disclosure.
FIG. 24 is a conceptual diagram of a mold used to manufacture a surface light source according to an embodiment of FIG. 19.
FIG. 25 is a table comparing a surface light source according to a third embodiment and a surface light source according to a fourth embodiment of the present disclosure with respect to the number of filaments and an amount of light depending on a distance between electrodes.

### [Mode for Invention]

Reference will now be made in detail to embodiments of the present disclosure, examples of which are illustrated in the accompanying drawings. Wherever possible, the same reference numbers will be used throughout the drawings to refer to the same or like parts.

In general, a suffix such as "assembly" and "unit" may be used to refer to elements or components. Use of such a suffix herein is merely intended to facilitate description of the present disclosure, and the suffix itself is not intended to give any special meaning or function.

It will be noted that a detailed description of known arts will be omitted if it is determined that the detailed description of the known arts can obscure embodiments of the present disclosure.

The accompanying drawings are used to help easily understand various technical features and it should be understood that embodiments presented herein are not limited by the accompanying drawings. As such, the present disclosure should be understood to extend to any alterations, equivalents and substitutes in addition to those which are particularly set out in the accompanying drawings.

The terms including an ordinal number such as first, second, etc. may be used to describe various components, but the components are not limited by such terms. The terms are used only for the purpose of distinguishing one component from other components.

When any component is described as "being coupled to" or "being in contact with" other component, this should be understood to mean that another component may exist between them although any component may be directly coupled to or in direct contact with the other component.

On the other hand, when any component is described as "being directly coupled to" or "being in direct contact with" other component, this should be understood to mean that no component exists between them.

A singular expression can include a plural expression as long as it does not have an apparently different meaning in context.

In the present disclosure, terms "include or comprise" or "have" should be understood to be intended to designate that illustrated features, numbers, steps, operations, components, parts or combinations thereof are present and not to preclude the existence of one or more other features, numbers, steps, operations, components, parts or combinations thereof, or the possibility of the addition thereof.

Reference will now be made in detail to embodiments of the present disclosure, examples of which are illustrated in the accompanying drawings. Wherever possible, the same reference numbers will be used throughout the drawings to refer to the same or like parts.

A far ultraviolet light emitting device according to embodiments of the present disclosure is described below.

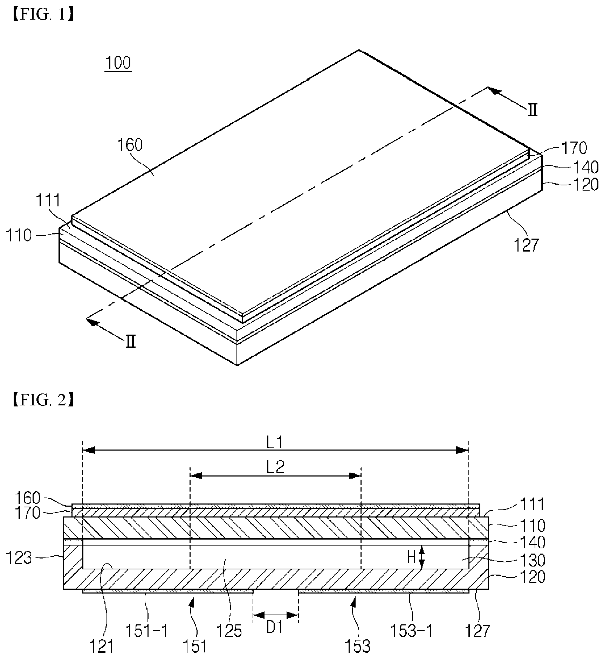

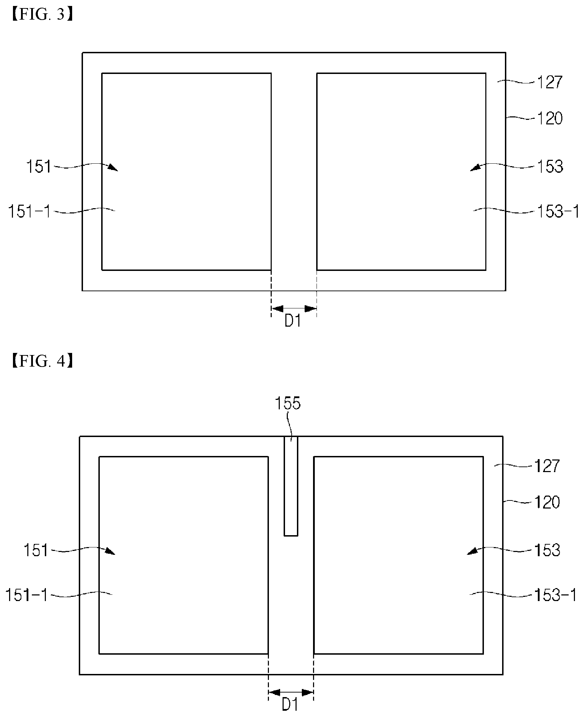

FIG. 1 is a perspective view of a surface light source according to a first embodiment of the present disclosure. FIG. 2 is a cross-sectional view taken along "II-II" of FIG. 1. FIG. 3 schematically illustrates an electrode structure included in the surface light source of FIG. 1.

A far ultraviolet light emitting device according to the present embodiment includes a surface light source 100 that emits light by a surface discharge.

The surface light source 100 includes a first plate member 110 having a light emitting surface 111 and a second plate member 120 that faces the first plate member 110 with a discharge space 130 interposed therebetween.

The first plate member 110 is formed of a flat plate, and the second plate member 120 is formed to be thicker than the first plate member 110. A concave portion 121 for forming the discharge space 130 is provided on an inner surface of the second plate member 120.

A first wall member 123 surrounding the concave portion 121 is provided at an edge of the inner surface of the second plate member 120.

The first wall member 123 may be seen as a remaining portion of the second plate member 120 except for the concave portion 121.

That is, the concave portion 121 and the first wall member 123 may be formed by removing a portion of the second plate member 120 thicker than the first plate member 110 by etching or the like, and the first wall members 123 may be viewed as a part of the second plate members 120. Thus, the first wall member 123 may be integrally formed with the second plate member 120.

The second plate member 120 may further include a second wall member 125 positioned in the discharge space 130.

The second wall member 125 may be formed using the same method as the first wall member 123 and may have a length L2 shorter than a length L1 of the discharge space 130.

The first plate member 110 and the second plate member 120 may be joined by a sealant 140. The sealant 140 may be positioned between the first wall member 123 and the first plate member 110 and may also be positioned between the second wall member 125 and the first plate members 110.

Therefore, since the second plate member 120 is joined to the first plate member 110 by the sealant 140, the discharge space 130 formed by the concave portion 121 of the second plate member120 can maintain a sealed state.

The first plate member 110 and the second plate member 120 may be made of quartz glass or ceramic.

Since each of the first wall member 123 and the second wall member 125 can be viewed as a part of the second plate member 120, the first wall member 123 and the second wall member 125 may also be made of quartz glass or ceramic.

The discharge space 130 may include an inert gas selected from the group consisting of argon (Ar), neon (Ne), xenon (Xe), and krypton (Kr), and the inert gas may be selected from the group consisting of ArBr, ArCl, ArF, ArO, NeF, XeI, XeO, XeBr, XeCl, XeF, KrBr, KrCl, KrO, and KrF.

An outer surface of the second plate member 120 includes an electrode surface 127, and a first electrode 151 and a second electrode 153 are disposed on the electrode surface 127.

As described above, in the surface light source 100 according to the present embodiment, the first electrode 151 and the second electrode 153 are disposed only on one surface of the surface light source 100, i.e., the electrode surface 127 of the second plate member 120, and are not disposed on the light emitting surface 111 of the first plate member 110.

Accordingly, the surface light source 100 according to the present embodiment emits light by a surface charge not a dielectric barrier discharge (DBD) formed when the first electrode 151 and the second electrode 153 are disposed on the different plate members 110 and 120.

According to the above-described configuration, since an acceleration distance of electrons and ions that generate discharge can be sufficiently secured without increasing a height H of the discharge space 130 formed between the first plate member 110 and the second plate member 120, the luminous efficiency of the surface light source 100 can be improved.

According to experiments conducted by the inventors, an amount of ultraviolet rays of a surface light source that emits light by dielectric barrier discharge was measured to be 0.8 mW/cm² to 2.64 mW/cm², but an amount of ultraviolet rays of the surface light source 100 according to the present embodiment that emits light by surface discharge was measured to be 6.2 mW/cm² to 11.2 mW/cm².

Accordingly, it can be seen that the luminous efficiency of the far ultraviolet light emitting device including the surface light source 100 according to the present embodiment that emits light by the surface discharge is much higher than luminous efficiency of a far ultraviolet light emitting device including the existing surface light source that emits light by the dielectric barrier discharge.

In addition, since the surface light source 100 according to the present embodiment can reduce the material cost of the electrodes 151 and 153, the manufacturing cost of the far ultraviolet light emitting device can be reduced.

That is, in the existing surface light source that emits light by the dielectric barrier discharge, i.e., a surface light source in which the first electrode and the second electrode are positioned opposite to each other, an electrode positioned on a light emitting surface is formed in a mesh type in order to secure the light emitting surface. In order to form the mesh type electrode, an electrode layer must be formed on the entire area of the light emitting surface, and then a portion of the electrode layer must be removed to form the mesh type electrode.

Therefore, there is a problem that the material cost of the electrode is high due to the electrode layer that is not used as an electrode and is removed.

However, in the present embodiment, since the electrode is not positioned on the light emitting surface 111, there is no need to form the first electrode 151 and the second electrode 153 in the mesh type. Further, although there may be some differences depending on an electrode formation method, an amount of electrode layer removed during the electrode formation process may be very small compared to the related art, or there may be no need to remove the electrode layer during the electrode formation process.

Accordingly, according to the present embodiment, since the material cost of the electrodes 151 and 153 can be reduced, the manufacturing cost of the far ultraviolet light emitting device can be reduced.

The first electrode 151 and the second electrode 153 disposed on the electrode surface 127 of the second plate member 120 may be formed in a symmetrical structure.

More specifically, in the present embodiment, the first electrode 151 may include a first main electrode portion 151-1, and the second electrode 153 may include a second main electrode portion 153-1.

The first main electrode portion 151-1 and the second main electrode portion 153-1 may be positioned to be spaced apart from each other at a first interval D1.

According to the above configuration, it is possible to appropriately control the acceleration distance of electrons or ions by appropriately setting a size of the first interval D1.

Each of the first main electrode portion 151-1 and the second main electrode portion 153-1 may be formed as a rectangular sheet electrode.

The first main electrode portion 151-1 and the second main electrode portion 153-1 may be formed on the electrode surface 127 by various known methods such as printing, coating, and sputtering.

FIGS. 4 to 6 illustrate various modifications in which the first electrode and the second electrode are formed in a symmetrical structure.

More specifically, first, as illustrated in FIG. 4, a surface light source according to a first modification may further include an auxiliary electrode 155 between the first main electrode portion 151-1 and the second main electrode portion 153-1, in addition to the first main electrode portion 151-1 and the second main electrode portion 153-1 illustrated in FIG. 3.

According to the above-described electrode structure further including the auxiliary electrode 155, a discharge inception voltage can be reduced compared to the embodiment of FIG. 3 described above.

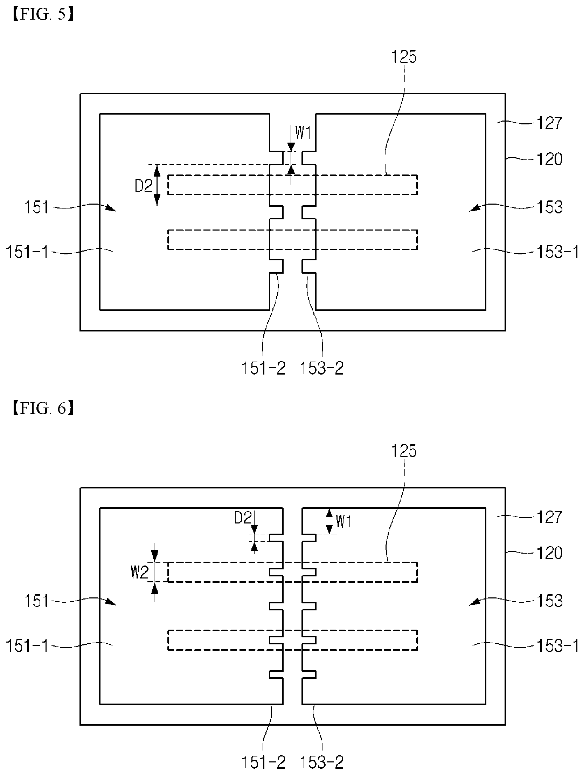

Next, as illustrated in FIG. 5, a surface light source according to a second modification may further include at least one first auxiliary electrode portion 151-2 and at least one second auxiliary electrode portion 153-2, in addition to the first main electrode portion 151-1 and the second main electrode portion 153-1 illustrated in FIG. 3.

The at least one first auxiliary electrode portion 151-2 may be integrally formed with the first main electrode portion 151-1 and may protrude toward the second main electrode portion 153-1.

The at least one second auxiliary electrode portion 153-2 may be integrally formed with the second main electrode portion 153-1 and may protrude toward the first main electrode portion 151-1.

The at least one first auxiliary electrode portion 151-2 and the at least one second auxiliary electrode portion 153-2 may face each other.

When the second plate member 120 includes the second wall member 125, the second wall member 125 may be disposed in a space between the adjacent auxiliary electrode portions as illustrated by the dotted line in FIG. 5.

For example, the second wall member 125 may be disposed between the adjacent first auxiliary electrode portions 151-2 and between the adjacent second auxiliary electrode portions 153-2.

The second wall member 125 may not overlap with the auxiliary electrode portions 151-2 and 153-2, or may partially overlap with the auxiliary electrode portions 151-2 and 153-2.

When the first auxiliary electrode portion 151-2 and the second auxiliary electrode portion 153-2 are provided, a discharge inception voltage can be reduced compared to the electrode structure according to the embodiment of FIG. 3.

According to the surface light source of the above-described configuration, when an alternating voltage is applied to the first electrode 151 and the second electrode 153, discharge filaments are formed in a number similar to the number of auxiliary electrode portions 151-2 and 153-2, and light emission occurs.

A width W1 of the first auxiliary electrode portion 151-2 and a width W1 of the second auxiliary electrode portion 153-2 may be less than an interval D2 between the auxiliary electrode portions.

Alternatively, as illustrated in FIG. 6, the width W1 of the first auxiliary electrode portion 151-2 and the width W1 of the second auxiliary electrode portion 153-2 may be greater than the interval D2 between the auxiliary electrode portions.

When the second plate member 120 includes the second wall member 125, the second wall member 125 may partially overlap with the auxiliary electrode portions 151-2 and 153-2 as illustrated by the dotted line in FIG. 6. However, the second wall member 125 may not overlap with the auxiliary electrode portions 151-2 and 153-2 by reducing a width W2 of the second wall member 125.

The above describes an example in which the first electrode and the second electrode are formed in a symmetrical structure, but the first electrode and the second electrode may be formed in an asymmetrical structure.

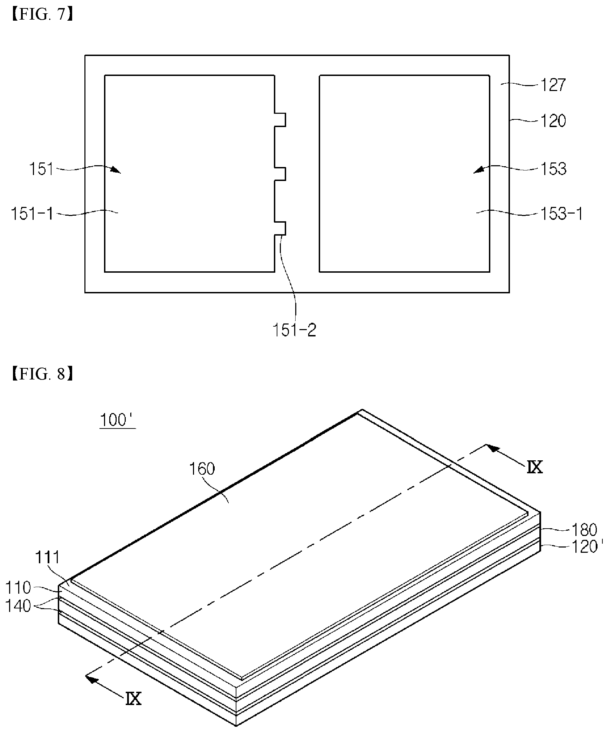

This is described with reference to FIG. 7. A surface light source according to a fourth modification may further include one auxiliary electrode portion of the first auxiliary electrode portion 151-2 and the second auxiliary electrode portion 153-2 illustrated in FIG. 5, in addition to the first main electrode portion 151-1 and the second main electrode portion 153-1 illustrated in FIG. 3.

FIG. 7 illustrates an example in which the first electrode 151 includes the first main electrode portion 151-1 and the first auxiliary electrode portion 151-2. However, the first electrode 151 may include only the first main electrode portion 151-1, and the second electrode 153 may include the second main electrode portion 153-1 and the second auxiliary electrode portion 153-2.

In the surface light source 100 including the electrodes with the above-described structure, as illustrated in FIGS. 1 and 2, an optical filter, for example, a bandpass filter 160, is disposed on the light emitting surface 111 of the first plate member 110.

The bandpass filter 160 substantially blocks ultraviolet rays in a wavelength range (240 nm or more and 300 nm or less) harmful to the human body, thereby enabling the killing of bacteria using only wavelengths of 190 nm to 240 nm, particularly 222 nm, that are harmless to the human body.

In the present embodiment, in order to dispose the bandpass filter 160 on the light emitting surface 111 of the surface light source 100, a fused silica substrate 170 may be used, and the bandpass filter 160 may be disposed on one surface, for example, an outer surface of the fused silica substrate 170.

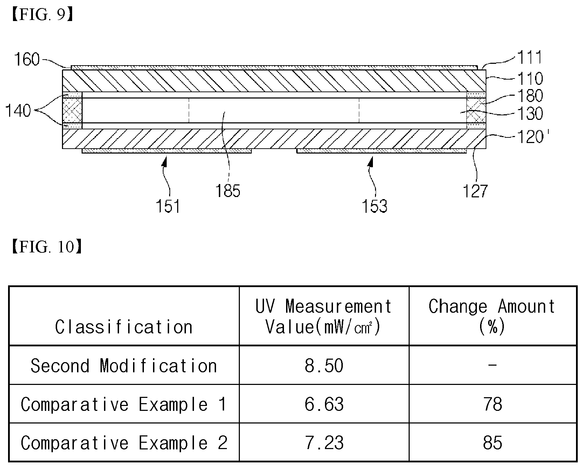

A surface light source 100' included in a far ultraviolet light emitting device according to a second embodiment of the present disclosure is described below with reference to FIGS. 8 and 9.

In the surface light source 100' according to the present embodiment, since an electrode structure can be formed in the same manner as any one of the first embodiment and the first to fourth modifications described above, a description of the electrode structure is omitted.

The first embodiment has described an example in which the second plate member 120 includes the concave portion 121, the first wall member 123, and the second wall member 125.

On the other hand, in the surface light source 100' according to the present embodiment, a second plate member 120' is formed of a flat plate in the same manner as a first plate member 110.

A first spacer 180 is disposed between the first plate member 110 and the second plate member 120'. The first spacer 180 is jointed to each of the first plate member 110 and the second plate member 120' by a sealant 140.

The first spacer 180 may be disposed at the same position as a first wall member 123. For example, the first spacer 180 may be disposed at an edge of the first plate member 110 and an edge of the second plate member 120'.

Accordingly, a discharge space 130 is formed between the first plate member 110 and the second plate member 120' by the first spacer 180.

As illustrated by the dotted line, a second spacer 185 may be further disposed in the discharge space 130.

The second spacer 185 acts in the same manner as the second wall member 125 of the first embodiment described above.

A bandpass filter 160 is formed directly on a light emitting surface 111 of the first plate member 110 without intervening the fused silica substrate 170.

The bandpass filter 160 may be directly coated on the light emitting surface 111 of the first plate member 110. Alternatively, the bandpass filter 160 may be attached to the light emitting surface 111 of the first plate member 110 by an adhesive.

According to the above configuration, since the fused silica substrate 170 of the first embodiment described above is not required, a reflection of some light at a boundary between the first plate member 110 and the fused silica substrate 170 can be reduced.

Therefore, light extraction efficiency can be increased, and the manufacturing cost of the far ultraviolet light emitting device can be reduced because the fused silica substrate is not used.

The structure that does not use the fused silica substrate can also be applied to the surface light source of the first embodiment described above.

FIG. 10 is a table for measuring an amount of ultraviolet rays of a surface light source having an electrode structure according to a second modification of the present disclosure, and the amount of ultraviolet rays is measured at a distance of 5 cm from the surface light source.

In FIG. 10, the second modification refers to a surface light source having the electrode structure illustrated in FIG. 5, a comparative example 1 refers to a surface light source applying the electrode structure of the second modification and the bandpass filter and the fused silica substrate of the first embodiment, and a comparative example 2 refers to a surface light source applying the electrode structure of the second modification and the bandpass filter of the second embodiment.

Referring to FIG. 10, in the surface light source having the electrode structure of the second modification, it can be seen that the amount of ultraviolet rays is more than in the comparative examples 1 and 2 because the bandpass filter is not applied.

However, in order to remove ultraviolet rays in a wavelength range harmless to the human body, the bandpass filter must be applied as in the comparative examples 1 and 2.

However, when examining the amount of ultraviolet rays in the comparative examples 1 and 2, it can be seen that the amount of ultraviolet rays in the comparative example 2 in which the bandpass filter is directly coated on the light emitting surface of the first plate member 110 is more than the amount of ultraviolet rays in the comparative example 1 further including the fused silica substrate.

The far ultraviolet light emitting device including the surface light source with the above configuration may further include a housing that accommodates the surface light source.

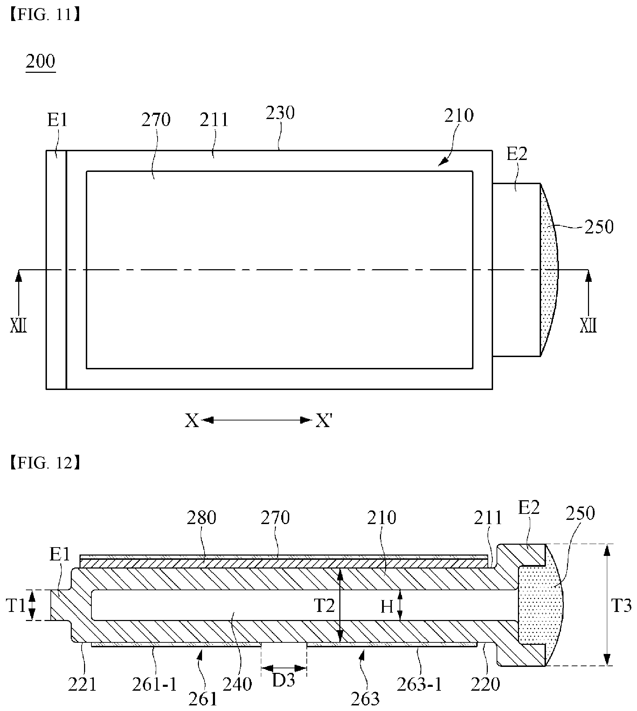

A surface light source 200 included in a far ultraviolet light emitting device according to a third embodiment of the present disclosure is described below with reference to FIGS. 11 to 18.

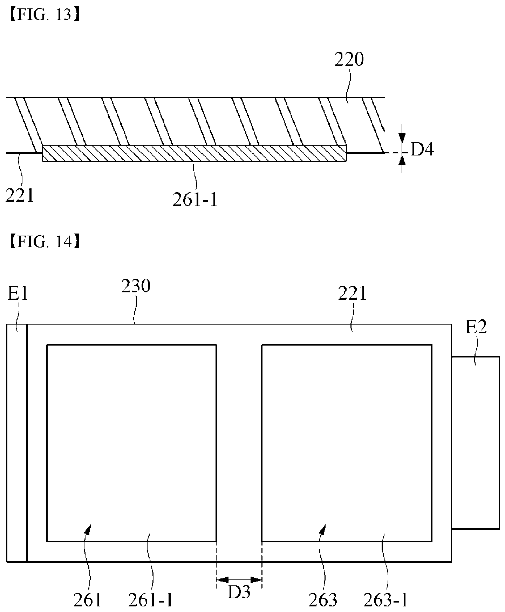

FIG. 11 is a plan view of a surface light source according to a third embodiment of the present disclosure. FIG. 12 is a cross-sectional view taken along "XII-XII" of FIG. 11. FIG. 13 is an enlarged view of main components of FIG. 12. FIG. 14 schematically illustrates an electrode structure included in the surface light source of FIG. 11.

The far ultraviolet light emitting device according to the third embodiment includes the surface light source 200 that emits light by a surface discharge.

The surface light source 200 includes a light emitting portion 210 including a light emitting surface 211, an electrode portion 220 facing the light emitting portion 210, and a side wall portion 230 connecting the light emitting portion 210 and the electrode portion 220 along a first direction X-X'.

The first direction X-X' may be a longitudinal direction of the surface light source 200. However, the first direction X-X' may be a width direction of the surface light source 200.

In the present embodiment, cross-sectional shapes of the light emitting portion 210 and the electrode portion 220 may be formed in a substantially flat plate shape, and a cross-sectional shape of the side wall portion 230 may be formed in an arc shape.

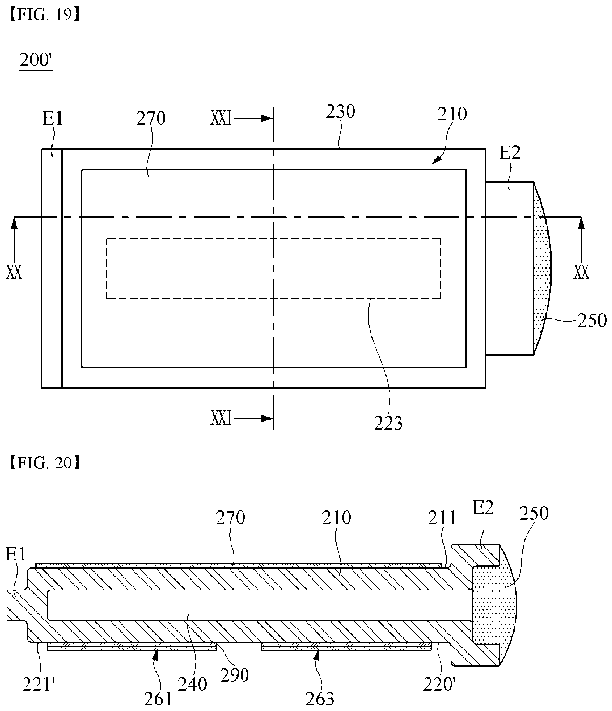

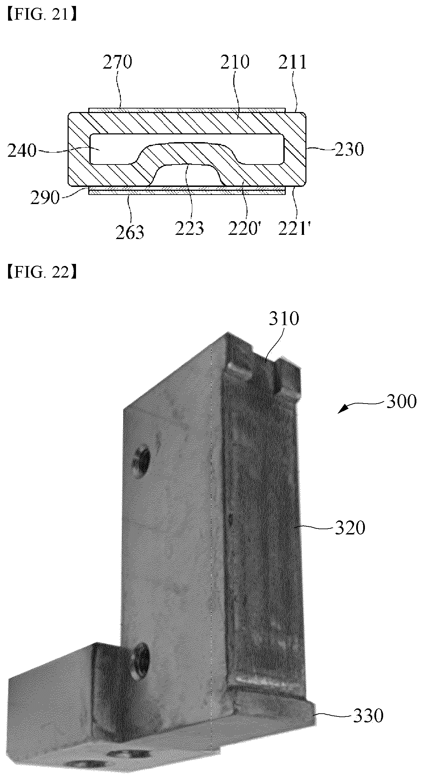

However, the cross-sectional shape of at least one of the light emitting portion 210 and the electrode portion 220 may be formed in a shape including a bent portion (see FIGS. 20 and 21), and the cross-sectional shape of the side wall portion 230 may be formed in a substantially flat plate shape.

As described above, the cross-sectional shapes of the light emitting portion 210, the electrode portion 220, and the side wall portion 230 may be changed by molds (see FIGS. 22 to 24) used in a manufacturing process of the surface light source 200.

A discharge space 240 is formed inside the surface light source 200 formed by the light emitting portion 210, the electrode portion 220, and the side wall portion 230.

The surface light source 200 includes a first end E1 and a second end E2 that are positioned on both sides in the first direction.

Referring to FIG. 11, the first end E1 refers to an end positioned on the left side, and the second end E2 refers to an end positioned on the right side.

The first end E1 may be formed as a joined end, and the second end E2 may be formed as a sealed end.

Here, the joined end refers to an end where two components, i.e., the light emitting portion 210 and the electrode portion 220 are joined in direct contact with each other, and the sealed end refers to an end where two components, i.e., the light emitting portion 210 and the electrode portion 220 are not joined in contact with each other, but extend in the first direction and are sealed by a separate sealant 250.

The joined end E1 may be formed in a shape with a flat rectangular cross-section, and the sealed end E2 may be formed in a shape with a circular cross-section.

According to the above configuration, a thickness T1 of the joined end E1 may be less than a thickness T2 of the surface light source 200, and a thickness T3 of the sealed end E2 may be greater than the thickness T2 of the surface light source 200.

Since the light emitting portion 210 and the electrode portion 220 are joined in direct contact with each other, the sealant 250 may not be provided between the light emitting portion 210 and the electrode portion 220 at the joined end E1. Hence, the light emitting portion 210 and the electrode portion 220 can be in direct contact with each other.

The surface light source 200 having the above shape may be manufactured using a mold 300 illustrated in FIG. 22. The mold 300 may be referred to as a "general mold".

A method of manufacturing the surface light source 200 is described below.

First, a cylindrical tube is heated using a hydrogen torch.

The cylindrical tube may be formed with an outer diameter of 10 mm to 20 mm and may be formed of a material such as quartz or borosilicate.

The cylindrical tube may be heated at a temperature of 1400 °C to 1600 °C for 5 to 30 seconds.

When the cylindrical tube is heated, the entire cylindrical tube may be heated while rotating the hydrogen torch, or only a portion of the cylindrical tube that is to be deformed using a mold may be heated. When the cylindrical tube is heated, the heated portion softens.

After the heating operation is completed, the cylindrical tube is formed using the mold 300.

The mold 300 may include an unformed portion 310 on which an unheated portion of the cylindrical tube is positioned, a first forming portion 320 for forming the light emitting portion 210 and/or the electrode portion 220 having the substantially flat shape, and a second forming portion 330 for forming the joined end E1.

When the cylindrical tube is formed, a pair of molds 300 may be positioned to face each other with the cylindrical tube interposed therebetween and may pressurize the cylindrical tube at the same pressure to form the cylindrical tube.

As above described, when the cylindrical tube is formed using the mold 300 at the outside of the cylindrical tube, it is possible to form the surface light source 200 with precise dimensions compared to the above-mentioned prior patent 2.

Here, the fact that the surface light source 200 has the precise dimensions means that the thickness of the light emitting portion 210, the thickness of the electrode portion 220, the thickness of the side wall portion 230, the height of the discharge space 240, etc. are individually and uniformly formed.

In order to improve the dimensional precision of the discharge space 240 and uniformly form the thicknesses of the light emitting portion 210 and the electrode portion 220, a compressed air may be injected into the inside of the cylindrical tube during the molding operation using the mold 300.

When the cylindrical tube is formed using the mold 300, the surface light source 200 including the substantially flat light emitting portion 210, the substantially flat electrode portion 220, the arc-shaped side wall portion 230, the first end E1, and the second end E2 may be manufactured.

As described above, the first end E1 may be formed as the joined end in which the light emitting portion 210 and the electrode portion 220 are in direct contact with each other to form a flat rectangular cross-sectional shape, and the second end E2 may be formed as the sealed end having a circular cross-sectional shape.

Therefore, the discharge space 240 can be maintained in a sealed state by forming the surface light source 200, then performing exhaust and gas injection operations through the second end E2, and then sealing the second end E2 using the sealant 250.

According to the above configuration, the surface light source according to the present embodiment does not need to form a separate exhaust tip because the unformed portion of the cylindrical tube, i.e., the second end E2 serves as an exhaust tip.

The light emitting portion 210, the electrode portion 220, the side wall portion 230, the first end E1, and the second end E2 may be made of quartz glass or ceramic.

The discharge space 240 may include an inert gas selected from the group consisting of argon (Ar), neon (Ne), xenon (Xe), and krypton (Kr), and the inert gas may be selected from the group consisting of ArBr, ArCl, ArF, ArO, NeF, XeI, XeO, XeBr, XeCl, XeF, KrBr, KrCl, KrO, and KrF.

An outer surface of the electrode portion 220 includes an electrode surface 221, and a first electrode 261 and a second electrode 263 are disposed on the electrode surface 221.

As described above, in the surface light source 200 according to the present embodiment, the first electrode 261 and the second electrode 263 are disposed only on one surface of the surface light source 200, i.e., the electrode surface 221 of the electrode portion 220, and are not disposed on the light emitting surface 211 of the light emitting portion 210.

Accordingly, the surface light source 200 according to the present embodiment emits light by a surface charge not a dielectric barrier discharge (DBD) formed when the first electrode 261 and the second electrode 263 are separately disposed on the different components, i.e., the light emitting portion 210 and the electrode portion 220.

According to the above-described configuration, since an acceleration distance of electrons and ions that generate discharge can be sufficiently secured without increasing a height H of the discharge space 240 formed between the light emitting portion 210 and the electrode portion 220, the luminous efficiency of the surface light source 200 can be improved.

According to experiments conducted by the inventors, an amount of ultraviolet rays of a surface light source that emits light by dielectric barrier discharge was measured to be 0.8 mW/cm² to 2.64 mW/cm², but an amount of ultraviolet rays of the surface light source 200 according to the present embodiment that emits light by surface discharge was measured to be 6.2 mW/cm² to 11.2 mW/cm².

Accordingly, it can be seen that the luminous efficiency of the far ultraviolet light emitting device including the surface light source 200 according to the present embodiment that emits light by the surface discharge is much higher than luminous efficiency of a far ultraviolet light emitting device including the existing surface light source that emits light by the dielectric barrier discharge.

In addition, since the surface light source 200 according to the present embodiment can reduce the material cost of the electrodes 261 and 263, the manufacturing cost of the far ultraviolet light emitting device can be reduced.

That is, in the existing surface light source that emits light by the dielectric barrier discharge, i.e., a surface light source in which the first electrode and the second electrode are positioned opposite to each other, an electrode positioned on a light emitting surface is formed in a mesh type in order to secure the light emitting surface. In order to form the mesh type electrode, an electrode layer must be formed on the entire area of the light emitting surface, and then a portion of the electrode layer must be removed to form the mesh type electrode.

Therefore, there is a problem that the material cost of the electrode is high due to the electrode layer that is not used as an electrode and is removed.

However, in the present embodiment, since the electrode is not positioned on the light emitting surface 211, there is no need to form the first electrode 261 and the second electrode 263 in the mesh type. Further, although there may be some differences depending on an electrode formation method, an amount of electrode layer removed during the electrode formation process may be very small compared to the related art, or there may be no need to remove the electrode layer during the electrode formation process.

Accordingly, according to the present embodiment, since the material cost of the electrodes 261 and 263 can be reduced, the manufacturing cost of the far ultraviolet light emitting device can be reduced.

The first electrode 261 and the second electrode 263 disposed on the electrode surface 221 of the electrode portion 220 may be formed in a symmetrical structure.

More specifically, in the present embodiment, the first electrode 261 may include a first main electrode portion 261-1, and the second electrode 263 may include a second main electrode portion 263-1.

The first main electrode portion 261-1 and the second main electrode portion 263-1 may be positioned to be spaced apart from each other at a first interval D3.

According to the above configuration, it is possible to appropriately control the acceleration distance of electrons or ions by appropriately setting a size of the first interval D3.

Each of the first main electrode portion 261-1 and the second main electrode portion 263-1 may be formed as a rectangular sheet electrode.

Alternatively, the first main electrode portion 261-1 and the second main electrode portion 263-1 may be formed in a stripe shape.

The first main electrode portion 261-1 and the second main electrode portion 263-1 may be formed on the electrode surface 221 by various known methods such as printing, coating, and sputtering.

In this case, the first electrode 261 and the second electrode 263 may be positioned on the electrode surface 221 of the electrode portion 220 as illustrated in FIG. 12.

Further, each of the first electrode 261 and the second electrode 263 may be formed as a metal thin plate.

In this case, in the molding operation using the mold 300, the first electrode 261 and the second electrode 263 may be disposed directly on the electrode surface 221 of the electrode portion 220, and a portion of each of the first electrode 261 and the second electrode 263 may be embedded in the electrode portion 220 as illustrated in FIG. 13.

That is, a portion of each of the first electrode 261 and the second electrode 263 may be embedded in the electrode portion 220 by a depth D4.

According to the above configuration, the first electrode 261 and the second electrode 263 can be firmly fixed to the electrode portion 220.

Further, even if each of the first electrode 261 and the second electrode 263 is formed as the metal thin plate, the first electrode 261 and the second electrode 263 may not be embedded in the electrode portion 220.

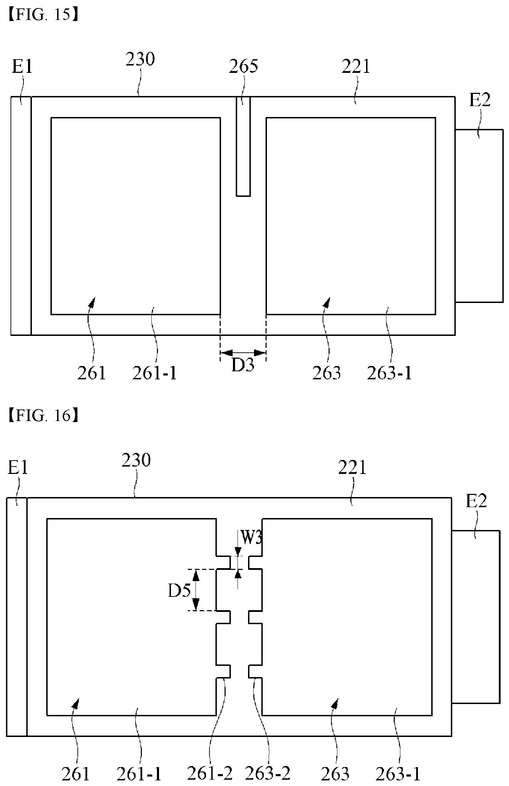

FIGS. 15 to 17 illustrate various modifications in which the first electrode and the second electrode are formed in a symmetrical structure.

More specifically, first, as illustrated in FIG. 15, a surface light source according to a first modification may further include an auxiliary electrode 265 between the first main electrode portion 261-1 and the second main electrode portion 263-1, in addition to the first main electrode portion 261-1 and the second main electrode portion 263-1 illustrated in FIG. 14.

According to the above-described electrode structure further including the auxiliary electrode 265, a discharge inception voltage can be reduced compared to the embodiment of FIG. 14 described above.

Next, as illustrated in FIG. 16, a surface light source according to a second modification may further include at least one first auxiliary electrode portion 261-2 and at least one second auxiliary electrode portion 263-2, in addition to the first main electrode portion 261-1 and the second main electrode portion 263-1 illustrated in FIG. 14.

The at least one first auxiliary electrode portion 261-2 may be integrally formed with the first main electrode portion 261-1 and may protrude toward the second main electrode portion 263-1.

The at least one second auxiliary electrode portion 263-2 may be integrally formed with the second main electrode portion 263-1 and may protrude toward the first main electrode portion 261-1.

The at least one first auxiliary electrode portion 261-2 and the at least one second auxiliary electrode portion 263-2 may face each other.

When the first auxiliary electrode portion 261-2 and the second auxiliary electrode portion 263-2 are formed, a discharge inception voltage can be reduced compared to the electrode structure of the embodiment illustrated in FIG. 14.

According to the surface light source of the above-described configuration, when an alternating voltage is applied to the first electrode 261 and the second electrode 263, discharge filaments are formed in a number similar to the number of auxiliary electrode portions 261-2 and 263-2, and light emission occurs.

A width W3 of the first auxiliary electrode portion 261-2 and a width W3 of the second auxiliary electrode portion 263-2 may be less than an interval D5 between the auxiliary electrode portions.

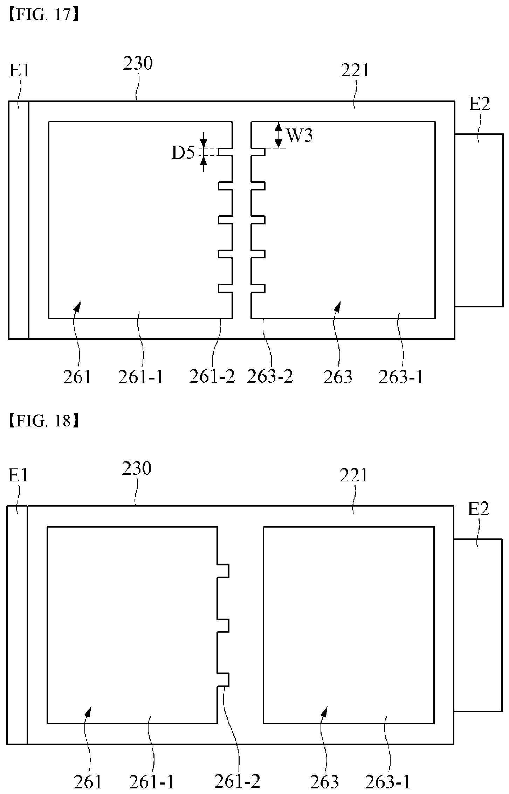

Alternatively, as illustrated in FIG. 17, the width W3 of the first auxiliary electrode portion 261-2 and the width W3 of the second auxiliary electrode portion 263-2 may be greater than the interval D5 between the auxiliary electrode portions.

The above describes an example in which the first electrode 261 and the second electrode 263 are formed in a symmetrical structure, but the first electrode 261 and the second electrode 263 may be formed in an asymmetrical structure.

This is described with reference to FIG. 18. A surface light source according to a fourth modification may further include one auxiliary electrode portion of the first auxiliary electrode portion 261-2 and the second auxiliary electrode portion 263-2 illustrated in FIG. 16, in addition to the first main electrode portion 261-1 and the second main electrode portion 263-1 illustrated in FIG. 14.

FIG. 18 illustrates an example in which the first electrode 261 includes the first main electrode portion 261-1 and the first auxiliary electrode portion 261-2. However, the first electrode 261 may include only the first main electrode portion 261-1, and the second electrode 263 may include the second main electrode portion 263-1 and the second auxiliary electrode portion 263-2.

In the surface light source 200 including the electrodes with the above-described structure, as illustrated in FIGS. 11 and 12, an optical filter, for example, a bandpass filter 270, is disposed on the light emitting surface 211 of the light emitting portion 210.

The bandpass filter 270 substantially blocks ultraviolet rays in a wavelength range (240 nm or more and 300 nm or less) harmful to the human body, thereby enabling the killing of bacteria using only wavelengths of 190 nm to 240 nm, particularly 222 nm, that are harmless to the human body.

In the present embodiment, in order to dispose the bandpass filter 270 on the light emitting surface 211 of the surface light source 200, a fused silica substrate 280 may be used, and the bandpass filter 270 may be disposed on one surface, for example, an outer surface of the fused silica substrate 280.

A surface light source 200' included in a far ultraviolet light emitting device according to a fourth embodiment of the present disclosure is described below with reference to FIGS. 19 and 21.

FIG. 19 is a plan view of a surface light source according to a fourth embodiment of the present disclosure. FIG. 20 is a cross-sectional view taken along "XX-XX" of FIG. 19. FIG. 21 is a cross-sectional view taken along "XXI-XXI" of FIG. 19.

In the surface light source 200' according to the present embodiment, since an electrode structure can be formed in the same manner as any one of the third embodiment and the first to fourth modifications described above, a description of the electrode structure is omitted.

The third embodiment has described an example in which the electrode portion 220 is formed in the substantially flat plate shape.

On the other hand, in the surface light source according to the present embodiment, an electrode portion 220' includes a bent portion 223.

Therefore, unlike the third embodiment described above, an electrode surface 221 of the electrode portion 220' includes a substantially flat portion and the bent portion 223.

According to the above-described configuration including the bent portion 223, since the number of discharge filaments with a high plasma density can be increased, an amount of ultraviolet rays can be increased, and stable discharge can be maintained.

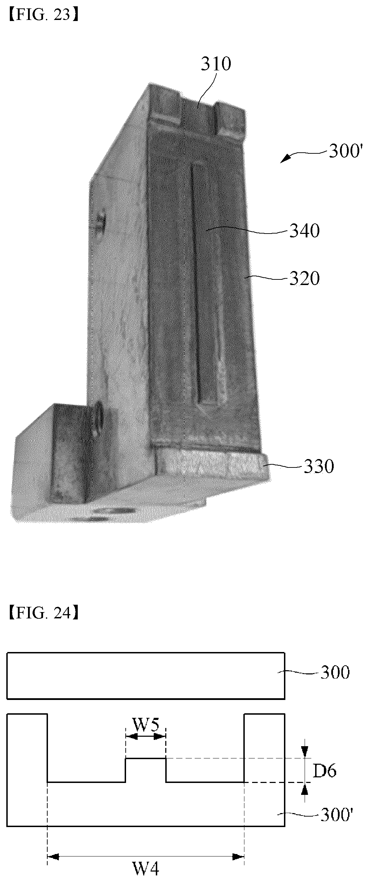

The electrode portion 220' including the bent portion 223 may be formed using a mold 300' illustrated in FIG. 23. The mold 300' may be referred to as a "special mold".

The mold 300' is entirely formed similarly to the mold 300 and further includes a third forming portion 340 for forming the bent portion 223.

The third forming portion 340 protrudes from a surface of a first forming portion 320. Therefore, when the mold 300' is used, the electrode portion 220' including the bent portion 223 can be formed.

The number and shape of the third forming portions 340 can be variously changed, and thus the number and shape of bent portions 223 can also be variously changed.

For example, the bent portion 223 may be formed in various cross-sectional shapes such as an arc shape, a trapezoidal shape, and a quadrangular shape.

The surface light source 200' according to the fourth embodiment may be formed using the molds 300 and 300' illustrated in FIG. 24.

Referring to FIG. 24, in the mold 300', a width W4 of the first forming portion 320 is 13 mm to 20 mm, a width W5 of the third forming portions 340 is 1 mm to 3 mm, and a thickness D6 of the third forming portions 340 is 0.3 mm to 1 mm.

Although not illustrated, when a pair of molds 300' are used, a surface light source in which each of a light emitting portion and an electrode portion includes a bent portion can be manufactured.

As described above, the bent portion may be included in at least one of the light emitting portion and the electrode portion.

In the present embodiment, a bandpass filter 270 may be formed directly on a light emitting surface 211 of a light emitting portion 210 without intervening the fused silica substrate 280.

The bandpass filter 270 may be directly coated on the light emitting surface 211 of the light emitting portion 210. Alternatively, the bandpass filter 270 may be attached to the light emitting surface 211 of the light emitting portion 210 by an adhesive.

According to the above configuration, since the fused silica substrate 280 of the third embodiment described above is not required, a reflection of some light at a boundary between the light emitting portion 210 and the fused silica substrate 280 can be reduced.

Therefore, light extraction efficiency can be increased, and the manufacturing cost of the far ultraviolet light emitting device can be reduced because the fused silica substrate is not used.

The structure that does not use the fused silica substrate can also be applied to the surface light source of the third embodiment described above.

In the present embodiment, the surface light source 200' may further include a reflective member 290 between the first electrode 261 and the electrode portion 220' and between the second electrode 263 and the electrode portion 220'.

The reflective member 290 may be formed of a PTFE material which is one of fluorine-based polymers with frail properties.

According to the above configuration, the electrodes 261 and 263 can be firmly attached to the reflective member 290 almost without being affected by the thickness non-uniformity of the electrode portion 220' and the thickness non-uniformity of the electrode material.

By arranging the reflective member 290 formed of a material having a higher reflectance than the electrode material between the electrodes 261 and 263 and the electrode portion 220', a loss of ultraviolet rays emitted from the surface light source 200' can be minimized, and the freedom of design of the electrode structure or the surface light source can be improved.

Each of the first electrode 261 and the second electrode 263 may be coated, printed, or deposited on the reflective member 290, and may be in direct contact with the surface of the reflective member 290.

According to the above configuration, after a cylindrical tube is formed, the reflective member 290 may be attached to the surface of the electrode portion 220', and then the first electrode 261 and the second electrode 263 may be formed directly on the surface of the reflective member 290.

Alternatively, each of the first electrode 261 and the second electrode 263 may be formed as a metal thin plate and may be in direct contact with the surface of the reflective member 290.

According to the above configuration, in the molding operation using the molds 300 and 300', the reflective member 290 may be disposed directly on the electrode portion 220'.

In this case, similar to the third embodiment illustrated in FIG. 13, a portion of the reflective member 290 may be embedded in the electrode portion 220'.

According to the above configuration, the reflective member 290 can be firmly fixed to the electrode portion 220'.

The above-described third embodiment and the above-described fourth embodiment can be combined in various ways.

For example, the electrode installation structure using the reflective member 290 can also be applied to the third embodiment described above.

In the same manner as this, the structure in which the electrode is formed directly on the electrode surface of the electrode portion without using the reflective member 290 can also be applied to the present embodiment.

The structure in which the bandpass filter is formed directly on the light emitting surface of the light emitting portion without using the fused silica substrate 280 can also be applied to the third embodiment described above.

In the same manner as this, the structure of the third embodiment in which the bandpass filter 270 is installed using the fused silica substrate 280 can also be applied to the fourth embodiment.

In addition, the number of filaments can be adjusted by variously adjusting the number of third forming portions 340 included in the mold 300' depending on the outer diameter size of the cylindrical tube.

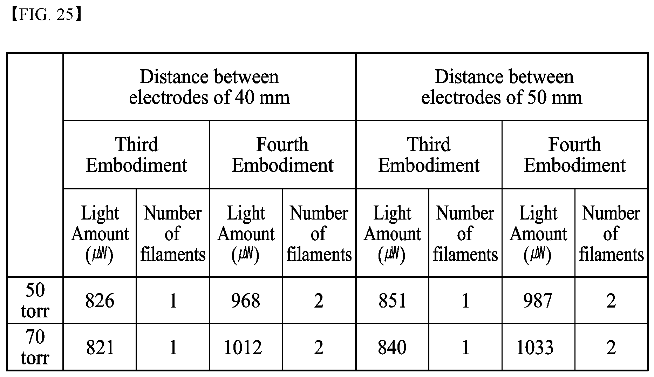

FIG. 25 is a table comparing the surface light source according to the third embodiment and the surface light source according to the fourth embodiment with respect to the number of filaments and an amount of light depending on a distance between electrodes.

Referring to FIG. 25, it can be seen that the surface light source of the fourth embodiment including the bent portion 223 has an increased number of filaments and an increased amount of light compared to the surface light source of the third embodiment not including the bent portion 223.

It can be seen that the amount of light when the distance between the first electrode 261 and the second electrode 263, i.e., the first interval D3 is 50 mm increases compared to when the first interval D3 is 40 mm. Further, it can be seen that the amount of light when the pressure of the discharge space 240 is 50 torr increases compared to when the pressure of the discharge space 240 is 70 torr.

The far ultraviolet light emitting device including the surface light source with the above-described configuration may further include a housing accommodating the surface light source.

It is apparent to those skilled in the art that the present disclosure can be embodied in other specific forms without departing from essential features of the present disclosure. Accordingly, the above detailed description should not be construed as limiting in all aspects and should be considered as illustrative. The scope of the present disclosure should be determined by rational construing of the appended claims, and all modifications within an equivalent scope of the present disclosure are included in the scope of the present disclosure.

## Claims

1. A far ultraviolet light emitting device comprising:
a surface light source configured to emit light by a surface discharge; and
a bandpass filter positioned on a light emitting surface of the surface light source,
wherein the surface light source includes:
a first plate member including the light emitting surface;
a second plate member configured to face the first plate member with a discharge space interposed therebetween, the second plate member including an outer surface including an electrode surface; and
a first electrode and a second electrode positioned on the electrode surface of the second plate member.

2. The far ultraviolet light emitting device of claim 1, wherein the first electrode includes a first main electrode portion, and the second electrode includes a second main electrode portion, and
wherein the first main electrode portion and the second main electrode portion are positioned to be spaced apart from each other at a first interval.

3. The far ultraviolet light emitting device of claim 2, wherein the first electrode and the second electrode are formed in an asymmetrical structure.

4. The far ultraviolet light emitting device of claim 3, wherein one of the first electrode and the second electrode includes at least one auxiliary electrode portion protruding toward the main electrode portion of the other electrode.

5. The far ultraviolet light emitting device of claim 2, wherein the first electrode and the second electrode are formed in a symmetrical structure.

6. The far ultraviolet light emitting device of claim 5, further comprising:
an auxiliary electrode positioned between the first main electrode portion and the second main electrode portion.

7. The far ultraviolet light emitting device of claim 5, wherein the first electrode further includes at least one first auxiliary electrode portion protruding toward the second main electrode portion, and
wherein the second electrode further includes at least one second auxiliary electrode portion protruding toward the first main electrode portion.

8. The far ultraviolet light emitting device of claim 7, wherein the at least one first auxiliary electrode portion and the at least one second auxiliary electrode portion face each other.

9. The far ultraviolet light emitting device of any one of claims 1 to 8, wherein the first plate member and the second plate member are quartz glass or ceramic.

10. The far ultraviolet light emitting device of claim 9, wherein the second plate member includes a concave portion for forming the discharge space and a first wall member that is positioned at an edge of the second plate member and surrounds the concave portion, and
wherein a sealant is positioned between the first wall member and the first plate member.

11. The far ultraviolet light emitting device of claim 10, wherein the second plate member further includes a second wall member positioned in the discharge space.

12. The far ultraviolet light emitting device of claim 10, wherein a fused silica substrate is positioned between the bandpass filter and the light emitting surface of the first plate member.

13. The far ultraviolet light emitting device of claim 10, wherein the bandpass filter is directly coated on the light emitting surface of the first plate member.

14. The far ultraviolet light emitting device of claim 10, wherein the discharge space includes an inert gas selected from a group including argon (Ar), neon (Ne), xenon (Xe), and krypton (Kr).

15. The far ultraviolet light emitting device of claim 9, further comprising:
a first spacer positioned between an edge of the first plate member and an edge of the second plate member,
wherein a sealant is positioned between the first spacer and the first plate member and between the first spacer and the second plate member.

16. The far ultraviolet light emitting device of claim 15, further comprising:
a second spacer positioned in the discharge space.

17. The far ultraviolet light emitting device of claim 15, wherein a fused silica substrate is positioned between the bandpass filter and the light emitting surface of the first plate member.

18. The far ultraviolet light emitting device of claim 15, wherein the bandpass filter is directly coated on the light emitting surface of the first plate member.

19. The far ultraviolet light emitting device of claim 15, wherein the discharge space includes an inert gas selected from a group including argon (Ar), neon (Ne), xenon (Xe), and krypton (Kr).

20. A far ultraviolet light emitting device comprising:
a surface light source configured to emit light by a surface discharge; and
a bandpass filter positioned on a light emitting surface of the surface light source,
wherein the surface light source includes:
a light emitting portion including the light emitting surface;
an electrode portion configured to face the light emitting portion with a discharge space interposed therebetween, the electrode portion including an electrode surface on which a first electrode and a second electrode are disposed; and
a side wall portion configured to connect the light emitting portion and the electrode portion along a first direction,
wherein a first end and a second end are positioned on both sides of the surface light source in the first direction,
wherein the first end is formed as a joined end at which the light emitting portion and the electrode portion are directly joined, and
wherein the second end is formed as a sealed end that extends from the light emitting portion and the electrode portion in the first direction and is sealed by a sealant.
